# EUROPEAN PATENT SPECIFICATION

(11) **EP 0 805 160 B1**
(45) Date of publication and mention of the grant of the patent: **07.05.2003**
(21) Application number: 97400985.4
(22) Date of filing: 30.04.1997
(51) Int. Cl.: C07K 2/00, C12N 15/62, G01N 33/53

(54) **Nucleic acid-bound polypeptide, method of producing nucleic acid-bound polypetide, and immuoassay using the polypeptide**
An Nukleinsäure gebundenes Polypeptid, Verfahren zur Herstellung eines an Nukleinsäure gebundenen Polypeptides und Immunoassay, in dem dieses Polypeptid Verwendung findet
Polypeptide lié à l'aide nucléique, procédé de préparation d'un polypeptide lié à l'acide nucléique et immunoessai utilisant le polypeptide

(30) Priority: 01.05.1996 JP 13444496
(43) Date of publication of application: 05.11.1997
(73) Proprietor: FUJIREBIO Inc., Shinjuku-ku, Tokyo 163-07 (JP)
(72) Inventor: Takemura, Fuminori, No. 806, Sakuragaoka Danchi 22, Higashiyamto-shi, Tokyo (JP); Ueno, Eiichi, Hino-shi, Tokyo (JP); Itoh, Satoru, 1-18-1, Narusegaoka, Machida-shi, Tokyo (JP)
(74) Representative: Uchida, Kenji

(56) References cited:
- EP-A- 0 704 221
- WO-A-93/14768
- JOURNAL OF VIROLOGY, vol. 64, no. 7, July 1990 F. BIRNBAUM et al. "Hepatitis B Virus Nucleocapsid Assem- bly: Primary Structure Re- quirements in the Core Pro- tein" pages 3319-3330
- Bonifer et al. (1989) J. Steroid Biochem. 32, 5-11.

### SEQUENCE LISTING

(1) GENERAL INFORMATION:
   (i) APPLICANT: Fuminori TAKEMURA et al.
   (ii) TITLE OF INVENTION: NUCLEIC ACID-BOUND POLYPEPTIDE, METHOD OF PRODUCIN G NUCLEIC ACID-BOUND POLYPEPTIDE, AND IMMUNOASSAY USING THE POLYPEPTIDE
   (iii)NUMBER OF SEQUENCES: 20
   (iv) CORRESPONDENCE ADDRESS:
      (A) ADDRESSEE: c/o FUJIREBIO INC., 7-1
      (B) STREET: Nishi-shinjuku 2-chome
      (C) CITY: Shinjuku-ku
      (D) STATE: Tokyo
      (E) COUNTRY: Japan
      (F) POSTAL CODE (ZIP): 163-07
   (v) COMPUTER READABLE FORM:
      (A) MEDIUM TYPE: Floppy disk
      (B) COMPUTER:NEC PC
      (C) OPERATING SYSTEM: MS-DOS
      (D) SOFTWARE:
   (vi) CURRENT APPLICATION DATA:
      (A) APPLICATION NUMBER:
      (B) FILING DATE:
   (vii)PRIOE APPLICATION DATA:
      (A) APPLICATION NUMBER: JP 134444/1996
      (B) FILING DATE: 1-MAY-1996
   (viii)ATTORNEY/AGENT INFORMATION
      (A) NAME:
      (B) REGISTRATION NUMBER:
      (C) REFERENCE/DOCKET NUMBER:
   (xi) TELECOMMUNICATION INFORMATION:
      (A) TELEPHONE:
      (B) TELEFAX:
(2) INFORMATION FOR SEQ ID NO: 1:
   (i) SEQUENCE CHARACTERISTICS:
      (A) LENGTH: 102 nucleic acids
      (B) TYPE: nucleic acid
      (C) STRANDEDNESS: single strand
      (D) TOPOLOGY: linear
   (ii) MOLECULE TYPE: other nucleic acid
   (vi) ORIGINAL SOURCE:
      (A) ORGANISM: synthesized
   (x) PUBLICATION INFORMATION:
      (A) AUTHORS: Fuminori TAKEMURA et al.
      (B) TITLE:
      (K) RELEVANT RESIDUES IN SEQ ID NO:1 : FROM 1 to 102
   (xi) SEQUENCE DESCRIPTION: SEQ ID NO:1 :
(2) INFORMATION FOR SEQ ID NO: 2:
   (i) SEQUENCE CHARACTERISTICS:
      (A) LENGTH: 34 amino acids
      (B) TYPE: amino acid
      (D) TOPOLOGY: linear
   (ii) MOLECULE TYPE: protein
   (vi) ORIGINAL SOURCE:
      (A) ORGANISM: recombinant
   (x) PUBLICATION INFORMATION:
      (A) AUTHORS: Fuminori TAKEMURA et al.
      (B) TITLE:
      (K) RELEVANT RESIDUES IN SEQ ID NO:2 : FROM 1 to 34
   (xi) SEQUENCE DESCRIPTION: SEQ ID NO:2 :
   Arg Arg Arg Gly Arg Ser Pro Arg Arg Arg Thr Pro Ser Pro Arg Arg
   1               5                   10                  15
   Arg Arg Ser Lys Ser Pro Arg Arg Arg Arg Ser Gln Ser Arg Glu Ser
               20                  25                  30
   Gln Cys
(2) INFORMATION FOR SEQ ID NO: 3:
   (i) SEQUENCE CHARACTERISTICS:
      (A) LENGTH: 360 nucleic acids
      (B) TYPE: nucleic acid
      (C) STRANDEDNESS: single strand
      (D) TOPOLOGY: linear
   (ii) MOLECULE TYPE: other nucleic acid
   (vi) ORIGINAL SOURCE:
      (A) ORGANISM: synthesized
   (x) PUBLICATION INFORMATION:
      (A) AUTHORS: Fuminori TAKEMURA et al.
      (B) TITLE:
      (K) RELEVANT RESIDUES IN SEQ ID NO:3 : FROM 1 to 360
   (xi) SEQUENCE DESCRIPTION: SEQ ID NO:3 :
(2) INFORMATION FOR SEQ ID NO: 4:
   (i) SEQUENCE CHARACTERISTICS:
      (A) LENGTH: 120 amino acids
      (B) TYPE: amino acid
      (D) TOPOLOGY: linear
   (ii) MOLECULE TYPE: protein
   (vi) ORIGINAL SOURCE:
      (A) ORGANISM: recombinant
   (x) PUBLICATION INFORMATION:
      (A) AUTHORS: Fuminori TAKEMURA et al.
      (B) TITLE:
      (K) RELEVANT RESIDUES IN SEQ ID NO:4 : FROM 1 to 120
   (xi) SEQUENCE DESCRIPTION: SEQ ID NO:4 :
   Met Ser Thr Asn Pro Lys Pro Gln Arg Lys Thr Lys Arg Asn Thr Asn
   1               5                   10                  15
   Arg Arg Pro Arg Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Pro Glu Gly Arg Thr Trp Ala Gln Pro Gly
   65                  70                  75                  80
   Tyr Pro Trp Pro Leu Tyr Gly Asn Glu Gly Met Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly
           115                 120
(2) INFORMATION FOR SEQ ID NO: 5:
   (i) SEQUENCE CHARACTERISTICS:
      (A) LENGTH: 450 nucleic acids
      (B) TYPE: nucleic acid
      (C) STRANDEDNESS: single strand
      (D) TOPOLOGY: linear
   (ii) MOLECULE TYPE: other nucleic acid
   (vi) ORIGINAL SOURCE:
      (A) ORGANISM: synthesized
   (x) PUBLICATION INFORMATION:
      (A) AUTHORS: Fuminori TAKEMURA et al.
      (B) TITLE:
      (K) RELEVANT RESIDUES IN SEQ ID NO:5 : FROM 1 to 450
   (xi) SEQUENCE DESCRIPTION: SEQ ID NO:5 :
(2) INFORMATION FOR SEQ ID NO: 6:
   (i) SEQUENCE CHARACTERISTICS:
      (A) LENGTH: 150 amino acids
      (B) TYPE: amino acid
      (D) TOPOLOGY: linear
   (ii) MOLECULE TYPE: protein
   (vi) ORIGINAL SOURCE:
      (A) ORGANISM: recombinant
   (x) PUBLICATION INFORMATION:
      (A) AUTHORS: Fuminori TAKEMURA et al.
      (B) TITLE:
      (K) RELEVANT RESIDUES IN SEQ ID NO:6 : FROM 1 to 150
   (xi) SEQUENCE DESCRIPTION: SEQ ID NO:6 :
   Met Ser Thr Asn Pro Lys Pro Gln Arg Lys Thr Lys Arg Asn Thr Asn
   1               5                   10                  15
   Arg Arg Pro Arg Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Pro Glu Gly Arg Thr Trp Ala Gln Pro Gly
   65                  70                  75                  80
   Tyr Pro Trp Pro Leu Tyr Gly Asn Glu Gly Met Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala
   145                 150
(2) INFORMATION FOR SEQ ID NO: 7:
   (i) SEQUENCE CHARACTERISTICS:
      (A) LENGTH: 483 nucleic acids
      (B) TYPE: nucleic acid
      (C) STRANDEDNESS: single strand
      (D) TOPOLOGY: linear
   (ii) MOLECULE TYPE: other nucleic acid
   (vi) ORIGINAL SOURCE:
      (A) ORGANISM: synthesized
   (x) PUBLICATION INFORMATION:
      (A) AUTHORS: Fuminori TAKEMURA et al.
      (B) TITLE:
      (K) RELEVANT RESIDUES IN SEQ ID NO:7 : FROM 1 to 483
   (xi) SEQUENCE DESCRIPTION: SEQ ID NO:7 :
(2) INFORMATION FOR SEQ ID NO: 8:
   (i) SEQUENCE CHARACTERISTICS:
      (A) LENGTH: 161 amino acids
      (B) TYPE: amino acid
      (D) TOPOLOGY: linear
   (ii) MOLECULE TYPE: protein
   (vi) ORIGINAL SOURCE:
      (A) ORGANISM: recombinant
   (x) PUBLICATION INFORMATION:
      (A) AUTHORS: Fuminori TAKEMURA et al.
      (B) TITLE:
      (K) RELEVANT RESIDUES IN SEQ ID NO:8 : FROM 1 to 161
   (xi) SEQUENCE DESCRIPTION: SEQ ID NO:8 :
   Met Ala Ser Glu Phe Met Ser Thr Asn Pro Lys Pro Gln Arg Lys Thr
   1               5                   10                  15
   Lys Arg Asn Thr Asn Arg Arg Pro Gln Asp Val Lys Phe Pro Gly Gly
                   20                  25                  30
   Gly Gln Ile Val Gly Gly Val Tyr Leu Leu Pro Arg Arg Gly Pro Arg
               35                  40                  45
   Leu Gly Val Arg Ala Thr Arg Lys Thr Ser Glu Arg Ser Gln Pro Arg
           50                  55                  60
   Gly Arg Arg Gln Pro Ile Pro Lys Ala Arg Arg Pro Glu Gly Arg Thr
   65                  70                  75                  80
   Trp Ala Gln Pro Gly Tyr Pro Trp Pro Leu Tyr Gly Asn Glu Gly Met
                       85                  90                  95
   Gly Trp Ala Gly Trp Leu Leu Ser Pro Arg Gly Ser Arg Pro Ser Trp
                   100                 105                 110
   Gly Pro Thr Asp Pro Arg Arg Arg Ser Arg Asn Leu Gly Gly Ser Arg
               115                 120                 125
   Arg Arg Gly Arg Ser Pro Arg Arg Arg Thr Pro Ser Pro Arg Arg Arg
           130                 135                 140
   Arg Ser Lys Ser Pro Arg Arg Arg Arg Ser Gln Ser Arg Glu Ser Gln
   145                 150                 155                 160
   Cys
(2) INFORMATION FOR SEQ ID NO: 9:
   (i) SEQUENCE CHARACTERISTICS:
      (A) LENGTH: 573 nucleic acids
      (B) TYPE: nucleic acid
      (C) STRANDEDNESS: single strand
      (D) TOPOLOGY: linear
   (ii) MOLECULE TYPE: other nucleic acid
   (vi) ORIGINAL SOURCE:
      (A) ORGANISM: synthesized
   (x) PUBLICATION INFORMATION:
      (A) AUTHORS: Fuminori TAKEMURA et al.
      (B) TITLE:
      (K) RELEVANT RESIDUES IN SEQ ID NO: 9 : FROM 1 to 573
   (xi) SEQUENCE DESCRIPTION: SEQ ID NO: 9 :
(2) INFORMATION FOR SEQ ID NO: 10:
   (i) SEQUENCE CHARACTERISTICS:
      (A) LENGTH: 191 amino acids
      (B) TYPE: amino acid
      (D) TOPOLOGY: linear
   (ii) MOLECULE TYPE: protein
   (vi) ORIGINAL SOURCE:
      (A) ORGANISM: recombinant
   (x) PUBLICATION INFORMATION:
      (A) AUTHORS: Fuminori TAKEMURA et al.
      (B) TITLE:
      (K) RELEVANT RESIDUES IN SEQ ID NO: 10 : FROM 1 to 191
   (xi) SEQUENCE DESCRIPTION: SEQ ID NO: 10:
   Met Ala Ser Glu Phe Met Ser Thr Asn Pro Lys Pro Gln Arg Lys Thr
   1 5 10 15
   Lys Arg Asn Thr Asn Arg Arg Pro Arg Asp Val Lys Phe Pro Gly Gly
   20 25 30
   Gly Gln Ile Val Gly Gly Val Tyr Leu Leu Pro Arg Arg Gly Pro Arg
   35 40 45
   Leu Gly Val Arg Ala Thr Arg Lys Thr Ser Glu Arg Ser Gln Pro Arg
   50 55 60
   Gly Arg Arg Gln Pro Ile Pro Lys Ala Arg Arg Pro Glu Gly Arg Thr
   65 70 75 80
   Trp Ala Gln Pro Gly Tyr Pro Trp Pro Leu Tyr Gly Asn Glu Gly Met
   85 90 95
   Gly Trp Ala Gly Trp Leu Leu Ser Pro Arg Gly Ser Arg Pro Ser Trp
   100 105 110
   Gly Pro Thr Asp Pro Arg Arg Arg Ser Arg Asn Leu Gly Lys Val Ile
   115 120 125
   Asp Thr Leu Thr Cys Gly Phe Ala Asp Leu Met Gly Tyr Ile Pro Leu
   130 135 140
   Val Gly Ala Pro Leu Gly Gly Ala Ala Arg Ala Gly Ser Arg Arg Arg
   145 150 155 160
   Gly Arg Ser Pro Arg Arg Arg Thr Pro Ser Pro Arg Arg Arg Arg Ser
   165 170 175
   Lys Ser Pro Arg Arg Arg Arg Ser Gln Ser Arg Glu Ser Gln Cys
   180 185 190
(2) INFORMATION FOR SEQ ID NO: 11:
   (i) SEQUENCE CHARACTERISTICS:
      (A) LENGTH: 843 nucleic acids
      (B) TYPE: nucleic acid
      (C) STRANDEDNESS: single strand
      (D) TOPOLOGY: linear
   (ii) MOLECULE TYPE: other nucleic acid
   (vi) ORIGINAL SOURCE:
      (A) ORGANISM: synthesized
   (x) PUBLICATION INFORMATION:
      (A) AUTHORS: Fuminori TAKEMURA et al.
      (B) TITLE:
      (K) RELEVANT RESIDUES IN SEQ ID NO:11 : PROM 1 to 843
   (xi) SEQUENCE DESCRIPTION: SEQ ID NO:11 :
(2) INFORMATION FOR SEQ ID NO: 12:
   (i) SEQUENCE CHARACTERISTICS:
      (A) LENGTH: 281 amino acids
      (B) TYPE: amino acid
      (D) TOPOLOGY: linear
   (ii) MOLECULE TYPE: protein
   (vi) ORIGINAL SOURCE:
      (A) ORGANISM: recombinant
   (x) PUBLICATION INFORMATION:
      (A) AUTHORS: Fuminori TAKEMURA et al.
      (B) TITLE:
      (K) RELEVANT RESIDUES IN SEQ ID NO:12 : FROM 1 to 281
   (xi) SEQUENCE DESCRIPTION: SEQ ID NO:12 :
   Met Ala Ser Met Ser Thr Asn Pro Lys Pro Gln Arg Lys Thr Lys Arg
   1 5 10 15
   Asn Thr Asn Arg Arg Pro Gln Asp Val Lys Phe Pro Gly Gly Gly Gln
   20 25 30
   Ile Val Gly Gly Val Tyr Leu Leu Pro Arg Arg Gly Pro Arg Leu Gly
   35 40 45
   Val Arg Ala Thr Arg Lys Thr Ser Glu Arg Ser Gln Pro Arg Gly Arg
   50 55 60
   Arg Gln Pro Ile Pro Lys Ala Arg Arg Pro Glu Gly Arg Thr Trp Ala
   65 70 75 80
   Gln Pro Gly Tyr Pro Trp Pro Leu Tyr Gly Asn Glu Gly Met Gly Trp
                       85                  90                  95
   Ala Gly Trp Leu Leu Ser Pro Arg Gly Ser Arg Pro Ser Trp Gly Pro
                   100                 105                 110
   Thr Asp Pro Arg Arg Arg Ser Arg Asn Leu Gly Glu Phe Arg Arg Arg
               115                 120                 125
   Gly Arg Ser Pro Arg Arg Arg Thr Pro Ser Pro Arg Arg Arg Arg Ser
       130                 135                 140
   Lys Ser Pro Arg Arg Arg Arg Ser Gln Ser Arg Glu Ser Gln Cys Glu
   145                 150                 155                 160
   Phe Met Ser Thr Asn Pro Lys Pro Gln Arg Lys Thr Lys Arg Asn Thr
                       165                 170                 175
   Asn Arg Arg Pro Gln Asp Val Lys Phe Pro Gly Gly Gly Gln Ile Val
                   180                 185                 190
   Gly Gly Val Tyr Leu Leu Pro Arg Arg Gly Pro Arg Leu Gly Val Arg
               195                 200                 205
   Ala Thr Arg Lys Thr Ser Glu Arg Ser Gln Pro Arg Gly Arg Arg Gln
       210                 215                 220
   Pro Ile Pro Lys Ala Arg Arg Pro Glu Gly Arg Thr Trp Ala Gln Pro
   225                 230                 235                 240
   Gly Tyr Pro Trp Pro Leu Tyr Gly Asn Glu Gly Met Gly Trp Ala Gly
                       245                 250                 255
   Trp Leu Leu Ser Pro Arg Gly Ser Arg Pro Ser Trp Gly Pro Thr Asp
                   260                 265                 270
   Pro Arg Arg Arg Ser Arg Asn Leu Gly
               275                 280
(2) INFORMATION FOR SEQ ID NO: 13:
   (i) SEQUENCE CHARACTERISTICS:
      (A) LENGTH: 1245 nucleic acids
      (B) TYPE: nucleic acid
      (C) STRANDEDNESS: single strand
      (D) TOPOLOGY: linear
   (ii) MOLECULE TYPE: other nucleic acid
   (vi) ORIGINAL SOURCE:
      (A) ORGANISM: synthesized
   (x) PUBLICATION INFORMATION:
      (A) AUTHORS: Fuminori TAKEMURA et al.
      (B) TITLE:
      (K) RELEVANT RESIDUES IN SEQ ID NO:13 : FROM 1 to 1245
   (xi) SEQUENCE DESCRIPTION: SEQ ID NO:13 :
(2) INFORMATION FOR SEQ ID NO: 14:
   (i) SEQUENCE CHARACTERISTICS:
      (A) LENGTH: 415 amino acids
      (B) TYPE: amino acid
      (D) TOPOLOGY: linear
   (ii) MOLECULE TYPE: protein
   (vi) ORIGINAL SOURCE:
      (A) ORGANISM: recombinant
   (x) PUBLICATION INFORMATION:
      (A) AUTHORS: Fuminori TAKEMURA et al.
      (B) TITLE:
      (K) RELEVANT RESIDUES IN SEQ ID NO:14 : FROM 1 to 415
   (xi) SEQUENCE DESCRIPTION: SEQ ID NO:14 :
   Met Gly Ser Ser His His Glu Thr His Tyr Gly Tyr Ala Thr Leu Ser
   1 5 10 15
   Tyr Ala Asp Tyr Trp Ala Gly Glu Leu Gly Gln Ser Arg Asp Val Leu
   20 25 30
   Leu Ala Gly Asn Ala Glu Ala Asp Arg Ala Gly Asp Leu Asp Ala Gly
   35 40 45
   Met Phe Asp Ala Val Ser Arg Ala Thr His Gly His Gly Ala Phe Arg
   50 55 60
   Gln Gln Phe Gln Tyr Ala Val Glu Val Leu Gly Glu Lys Val Leu Ser
   65 70 75 80
   Lys Gln Glu Thr Glu Asp Ser Arg Gly Arg Lys Lys Trp Glu Tyr Glu
   85 90 95
   Thr Asp Pro Ser Val Thr Lys Met Val Arg Ala Ser Ala Ser Phe Gln
   100 105 110
   Asp Leu Gly Glu Asp Gly Glu Ile Lys Phe Glu Ala Val Glu Gly Ala
   115 120 125
   Val Ala Leu Ala Asp Arg Ala Ser Ser Phe Met Val Asp Ser Glu Glu
   130 135 140
   Tyr Lys Ile Thr Asn Val Lys Val His Gly Met Lys Phe Val Pro Val
   145 150 155 160
   Ala Val Pro His Glu Leu Lys Gly Ile Ala Lys Glu Lys Phe His Phe
   165 170 175
   Val Glu Asp Ser Arg Val Thr Glu Asn Thr Asn Gly Leu Lys Thr Met
   180 185 190
   Leu Thr Glu Asp Ser Phe Ser Ala Arg Lys Val Ser Ser Met Glu Ser
           195                 200                 205
   Pro His Asp Leu Val Val Asp Thr Val Gly Thr Val Tyr His Ser Arg
       210                 215                 220
   Phe Gly Ser Asp Ala Glu Ala Ser Val Met Leu Lys Arg Ala Asp Gly
   225                 230                 235                 240
   Ser Glu Leu Ser His Arg Glu Phe Ile Asp Tyr Val Met Asn Phe Asn
                   245                 250                 255
   Thr Val Arg Tyr Asp Tyr Tyr Gly Asp Asp Ala Ser Tyr Thr Asn Leu
               260                 265                 270
   Met Ala Ser Tyr Gly Thr Lys His Ser Ala Asp Ser Trp Trp Lys Thr
           275                 280                 285
   Gly Arg Val Pro Arg Ile Ser Cys Gly Ile Asn Tyr Gly Phe Asp Arg
       290                 295                 300
   Phe Lys Gly Ser Gly Pro Gly Tyr Tyr Arg Leu Thr Leu Ile Ala Asn
   305                 310                 315                 320
   Gly Tyr Arg Asp Val Val Ala Asp Val Arg Phe Leu Pro Lys Tyr Glu
                   325                 330                 335
   Gly Asn Ile Asp Ile Gly Leu Lys Gly Lys Val Leu Thr Ile Gly Gly
               340                 345                 350
   Ala Asp Ala Glu Thr Leu Met Asp Ala Ala Val Asp Val Phe Ala Asp
           355                 360                 365
   Gly Gln Pro Lys Leu Val Ser Asp Gln Ala Val Ser Leu Gly Gln Asn
       370                 375                 380
   Val Leu Ser Ala Asp Phe Thr Pro Gly Thr Glu Tyr Thr Val Glu Val
   385                 390                 395                 400
   Arg Phe Lys Glu Phe Gly Ser Val Arg Ala Lys Val Val Ala Gln
                   405                 410                 415
(2) INFORMATION FOR SEQ ID NO: 15:
   (i) SEQUENCE CHARACTERISTICS:
      (A) LENGTH: 1368 nucleic acids
      (B) TYPE: nucleic acid
      (C) STRANDEDNESS: single strand
      (D) TOPOLOGY: linear
   (ii) MOLECULE TYPE: other nucleic acid
   (vi) ORIGINAL SOURCE:
      (A) ORGANISM: synthesized
   (x) PUBLICATION INFORMATION:
      (A) AUTHORS: Fuminori TAKEMURA et al.
      (B) TITLE:
      (K) RELEVANT RESIDUES IN SEQ ID NO:15 : FROM 1 to 1368
   (xi) SEQUENCE DESCRIPTION: SEQ ID NO:15 :
(2) INFORMATION FOR SEQ ID NO: 16:
   (i) SEQUENCE CHARACTERISTICS:
      (A) LENGTH: 456 amino acids
      (B) TYPE: amino acid
      (D) TOPOLOGY: linear
   (ii) MOLECULE TYPE: protein
   (vi) ORIGINAL SOURCE:
      (A) ORGANISM: recombinant
   (x) PUBLICATION INFORMATION:
      (A) AUTHORS: Fuminori TAKEMURA et al.
      (B) TITLE:
      (K) RELEVANT RESIDUES IN SEQ ID NO:16 : FROM 1 to 456
   (xi) SEQUENCE DESCRIPTION: SEQ ID NO:16 :
   Met Ala Ser Glu Phe Met Gly Ser Ser His His Glu Thr His Tyr Gly
   1               5                   10                  15
   Tyr Ala Thr Leu Ser Tyr Ala Asp Tyr Trp Ala Gly Glu Leu Gly Gln
               20                  25                  30
   Ser Arg Asp Val Leu Leu Ala Gly Asn Ala Glu Ala Asp Arg Ala Gly
           35                  40                  45
   Asp Leu Asp Ala Gly Met Phe Asp Ala Val Ser Arg Ala Thr His Gly
       50                  55                  60
   His Gly Ala Phe Arg Gln Gln Phe Gln Tyr Ala Val Glu Val Leu Gly
   65                  70                  75                  80
   Glu Lys Val Leu Ser Lys Gln Glu Thr Glu Asp Ser Arg Gly Arg Lys
                   85                  90                  95
   Lys Trp Glu Tyr Glu Thr Asp Pro Ser Val Thr Lys Met Val Arg Ala
               100                 105                 110
   Ser Ala Ser Phe Gln Asp Leu Gly Glu Asp Gly Glu Ile Lys Phe Glu
           115                 120                 125
   Ala Val Glu Gly Ala Val Ala Leu Ala Asp Arg Ala Ser Ser Phe Met
   130 135 140
   Val Asp Ser Glu Glu Tyr Lys Ile Thr Asn Val Lys Val His Gly Met
   145 150 155 160
   Lys Phe Val Pro Val Ala Val Pro His Glu Leu Lys Gly Ile Ala Lys
   165 170 175
   Glu Lys Phe His Phe Val Glu Asp Ser Arg Val Thr Glu Asn Thr Asn
   180 185 190
   Gly Leu Lys Thr Met Leu Thr Glu Asp Ser Phe Ser Ala Arg Lys Val
   195 200 205
   Ser Ser Met Glu Ser Pro His Asp Leu Val Val Asp Thr Val Gly Thr
   210 215 220
   Val Tyr His Ser Arg Phe Gly Ser Asp Ala Glu Ala Ser Val Met Leu
   225 230 235 240
   Lys Arg Ala Asp Gly Ser Glu Leu Ser His Arg Glu Phe Ile Asp Tyr
   245 250 255
   Val Met Asn Phe Asn Thr Val Arg Tyr Asp Tyr Tyr Gly Asp Asp Ala
   260 265 270
   Ser Tyr Thr Asn Leu Met Ala Ser Tyr Gly Thr Lys His Ser Ala Asp
   275 280 285
   Ser Trp Trp Lys Thr Gly Arg Val Pro Arg Ile Ser Cys Gly Ile Asn
   290 295 300
   Tyr Gly Phe Asp Arg Phe Lys Gly Ser Gly Pro Gly Tyr Tyr Arg Leu
   305 310 315 320
   Thr Leu Ile Ala Asn Gly Tyr Arg Asp Val Val Ala Asp Val Arg Phe
   325 330 335
   Leu Pro Lys Tyr Glu Gly Asn Ile Asp Ile Gly Leu Lys Gly Lys Val
   340 345 350
   Leu Thr Ile Gly Gly Ala Asp Ala Glu Thr Leu Met Asp Ala Ala Val
   355 360 365
   Asp Val Phe Ala Asp Gly Gln Pro Lys Leu Val Ser Asp Gln Ala Val
   370 375 380
   Ser Leu Gly Gln Asn Val Leu Ser Ala Asp Phe Thr Pro Gly Thr Glu
   385 390 395 400
   Tyr Thr Val Glu Val Arg Phe Lys Glu Phe Gly Ser Val Arg Ala Lys
   405 410 415
   Val Val Ala Gln Gly Ser Arg Arg Arg Gly Arg Ser Pro Arg Arg Arg
   420 425 430
   Thr Pro Ser Pro Arg Arg Arg Arg Ser Lys Ser Pro Arg Arg Arg Arg
   435 440 445
   Ser Gln Ser Arg Glu Ser Gln Cys
   450 455
(2) INFORMATION FOR SEQ ID NO: 17:
   (i) SEQUENCE CHARACTERISTICS:
      (A) LENGTH: 153 nucleic acids
      (B) TYPE: nucleic acid
      (C) STRANDEDNESS: single strand
      (D) TOPOLOGY: linear
   (ii) MOLECULE TYPE: cDNA
   (vi) ORIGINAL SOURCE:
      (A) ORGANISM: synthesized
   (x) PUBLICATION INFORMATION:
      (A) AUTHORS: Fuminori TAKEMURA et al.
      (B) TITLE:
      (K) RELEVANT RESIDUES IN SEQ ID NO:17 : FROM 1 to 153
   (xi) SEQUENCE DESCRIPTION: SEQ ID NO:17 :
(2) INFORMATION FOR SEQ ID NO: 18:
   (i) SEQUENCE CHARACTERISTICS:
      (A) LENGTH: 51 amino acids
      (B) TYPE: amino acid
      (D) TOPOLOGY: linear
   (ii) MOLECULE TYPE: protein
   (vi) ORIGINAL SOURCE:
      (A) ORGANISM: recombinant
   (x) PUBLICATION INFORMATION:
      (A) AUTHORS: Fuminori TAKEMURA et al.
      (B) TITLE:
      (K) RELEVANT RESIDUES IN SEQ ID NO:18 : FROM 1 to 51
   (xi) SEQUENCE DESCRIPTION: SEQ ID NO:18 :
   Met Ala Arg Tyr Arg Cys Cys Arg Ser Lys Ser Arg Ser Arg Cys Arg
   1               5                   10                  15
   Arg Arg Arg Arg Arg Cys Arg Arg Arg Arg Arg Arg Cys Cys Arg Arg
               20                  25                  30
   Arg Arg Arg Arg Cys Cys Arg Arg Arg Arg Ser Tyr Thr Ile Arg Cys
           35                  40                  45
   Lys Lys Tyr
       50
(2) INFORMATION FOR SEQ ID NO: 19:
   (i) SEQUENCE CHARACTERISTICS:
      (A) LENGTH: 528 nucleic acids
      (B) TYPE: nucleic acid
      (C) STRANDEDNESS: single strand
      (D) TOPOLOGY: linear
   (ii) MOLECULE TYPE: other nucleic acid
   (vi) ORIGINAL SOURCE:
      (A) ORGANISM: synthesized
   (x) PUBLICATION INFORMATION:
      (A) AUTHORS: Fuminori TAKEMURA et al.
      (B) TITLE:
      (K) RELEVANT RESIDUES IN SEQ ID NO:19 : FROM 1 to 528
   (xi) SEQUENCE DESCRIPTION: SEQ ID NO:19 :
(2) INFORMATION FOR SEQ ID NO: 20:
   (i) SEQUENCE CHARACTERISTICS:
      (A) LENGTH: 176 amino acids
      (B) TYPE: amino acid
      (D) TOPOLOGY: linear
   (ii) MOLECULE TYPE: protein
   (vi) ORIGINAL SOURCE:
      (A) ORGANISM: recombinant
   (x) PUBLICATION INFORMATION:
      (A) AUTHORS: Fuminori TAKEMURA et al.
      (B) TITLE:
      (K) RELEVANT RESIDUES IN SEQ ID NO:20 : FROM 1 to 176
   (xi) SEQUENCE DESCRIPTION: SEQ ID NO:20 :
   Met Ala Ser Met Ser Thr Asn Pro Lys Pro Gln Arg Lys Thr Lys Arg
   1               5                   10                  15
   Asn Thr Asn Arg Arg Pro Arg Asp Val Lys Phe Pro Gly Gly Gly Gln
               20                  25                  30
   Ile Val Gly Gly Val Tyr Leu Leu Pro Arg Arg Gly Pro Arg Leu Gly
           35                  40                  45
   Val Arg Ala Thr Arg Lys Thr Ser Glu Arg Ser Gln Pro Arg Gly Arg
       50                  55                  60
   Arg Gln Pro Ile Pro Lys Ala Arg Arg Pro Glu Gly Arg Thr Trp Ala
   65                  70                  75                  80
   Gln Pro Gly Tyr Pro Trp Pro Leu Tyr Gly Asn Glu Gly Met Gly Trp
                   85                  90                  95
   Ala Gly Trp Leu Leu Ser Pro Arg Gly Ser Arg Pro Ser Trp Gly Pro
               100                 105                 110
   Thr Asp Pro Arg Arg Arg Ser Arg Asn Leu Gly Glu Phe Met Ala Arg
           115                 120                 125
   Tyr Arg Cys Cys Arg Ser Lys Ser Arg Ser Arg Cys Arg Arg Arg Arg
       130                 135                 140
   Arg Arg Cys Arg Arg Arg Arg Arg Arg Cys Cys Arg Arg Arg Arg Arg
   145                 150                 155                 160
   Arg Cys Cys Arg Arg Arg Arg Ser Tyr Thr Ile Arg Cys Lys Lys Tyr
                   165                 170                 175

## Description

### BACKGROUND OF THE INVENTION

### Field of the Invention

The present invention relates to an immunoassay using the nucleic acid-bound polypeptide.

### Discussion of Background

Various studies have been made as to how to maintain the specific steric structure of a recombinant protein produced by gene engineering, more specifically gene manipulation, and also as to how to apply the thus produced protein to an antigen-antibody reaction.

In the production of the recombinant protein, in particular, in the course of a purification step of the produced protein, a denaturation operation is inevitably carried out. In such purification step, it is not always possible to maintain a natural structure of the protein, so that such protein cannot be used in an immunoassay system.

Various factors are also known that affect reactions which are peculiar to each of various assays. It is known that for these reasons or other, the above-mentioned antigen-antibody reaction does not always proceed as desired when the recombinant protein is used.

For example, there is known an agglutination immunoassay as one of immunoassays. For instance, when an antibody corresponding to an antigen is assayed by agglutination immunoassay, the antigen is fixed on the surface of particles such as latex particles, and such antigen-fixed particles are allowed to react with the antibody in a test sample. When the antibody is present in the test sample, the antigen-fixed particles agglutinate due to the antigen-antibody reaction, so that, for instance, the absorbance of the test sample changes. Therefore by measuring the absorbance of the test sample, the degree of the agglutination can be determined, and accordingly the antibody in the test sample can be quantitatively measured from the measured absorbance of the test sample.

However, when the recombinant protein is used as the antigen to be fixed on the surface of the particles in the above-mentioned agglutination immunoassay, it occasionally occurs that even though the protein itself has reactivity with the antibody to be assayed and the antibody is in fact present in the test sample, no agglutination takes place.

Conventionally, in the case where no agglutination takes place as mentioned above, the recombinant protein is modified or expressed in the form of a fused protein in order to improve the agglutination reactivity of the protein. However, it is extremely difficult to modify the protein so as to impart the desired properties thereto, while maintaining the antigenicity (i.e. the reactivity with the antibody).

Furthermore, the recombinant protein is often of an insoluble kind, so that when the thus produced protein is purified, the protein has to be subjected to solubilization treatment. However, the protein is often denatured in the course of the purification treatment, losing the necessary antigenicity.

Therefore, it is preferable that a soluble protein be directly produced by genetic engineering.

### SUMMARY OF THE INVENTION

The object of the present invention can be achieved by an immunoassay for assaying an antigen comprising a polypeptide, or an antibody corresponding to the antigen, using as the antigen the above-mentioned wherein said nucleic acid-bound polypeptide is a fusion polypeptide comprising a polypeptide with a nucleic acid-bounding motif including the amino acid sequence shown in SEQ ID NO 2 , whereto said nucleic acid is bound.

The above nucleic acid-bound polypeptide can be produced by the method comprising the steps of:
producing a recombinant polypeptide,
binding a nucleic acid to the recombinant polypeptide to produce a nucleic acid-bound polypeptide as a soluble fraction, and
purifying the nucleic acid-bound polypeptide from the soluble fraction.

In the above-mentioned method of producing the nucleic acid-bound polypeptide, the step of binding the nucleic acid to the polypeptide to produce the nucleic acid-bound polypeptide may comprise the steps of:
fusing a gene which encodes the polypeptide and a gene which encodes the nucleic acid-binding motif to produce a fusion gene, and
expressing the fusion gene to produce the nucleic acid-bound polypeptide via the nucleic acid-binding motif.

### BRIEF DESCRIPTION OF THE DRAWINGS

A more complete appreciation of the invention and many of the attendant advantages thereof will be readily obtained as the same becomes better understood by reference to the following detailed description when considered in connection with the accompanying drawings, wherein:
Fig. 1 is a genetic map of a cloning vector pW6A for use in expressing HCV core protein used in the examples of the present invention.
Fig. 2 is a diagram showing the results of Western blotting performed for showing the reactivity of an HCV core protein prepared by genetic engineering in an example of the present invention with HCV core positive human serum.

### DESCRIPTION OF THE PREFERRED EMBODIMENTS

The nucleic acid-bound polypeptide used in the immuno assay of the present invention can be provided by binding a nucleic acid to a polypeptide, whereby the properties of the polypeptide, such as the isoelectric point, the molecular weight and the three-dimensional structure thereof, can be changed without the antigenicity thereof being changed.

As the "polypeptide" for use in the present invention, any polypeptide can be employed as long as the polypeptide itself exhibits antigenicity and therefore the number of amino acid residues which constitute the polypeptide is 6 or more. It is preferable that the number of the amino acid residues which constitute the "polypeptide" for use in the present invention be 8 or more.

Examples of the "polypeptide" for use in the present invention include composites of a polypeptide and another component or other components such as sugar or lipid, namely glycoprotein and lipoprotein.

There is no particular limitation to the size of the nucleic acid which is bound to the polypeptide as long as the nucleic acid can change the above-mentioned properties of the polypeptide, such as isoelectric point, molecular weight and three-dimensional structure, without changing the antigenicity thereof. Normally, the number of bases of the nucleic acid for use in the present invention is 100 b to 10 kb, preferably about 1 kb to 5 kb.

Furthermore, the nucleic acid to be bound to the polypeptide may be either DNA or RNA.

The nucleic acid may be bonded to any portion of the polypeptide. For instance, the nucleic acid may be bonded to the N-terminus or the C-terminus of the polypeptide, but the bonding is not limited to such terminus. In the present invention, the nucleic acid may be either directly or indirectly bonded to the polypeptide. For instance, the nucleic acid may be bonded to the polypeptide via a nucleic acid-binding motif which is also a polypeptide.

In this application, with respect to the binding of the nucleic acid to the polypeptide, the term "binding" or "bound" means all kinds of chemical bondings between the polypeptide and the nucleic acid with attractive force in a wide range of relatively weak attractive force to strong attractive force, without any particular limitation to the bonding mode, including the so-called association, covalent bonding, ionic bonding, coordinate bonding, and hydrogen bonding.

In the present invention, when the nucleic acid-bound polypeptide is produced by genetic engineering, the nucleic acid-bound polypeptide may be expressed in the form of a polypeptide to which the nucleic acid is bound, thereby producing the nucleic acid-bound polypeptide. Alternatively, after a recombinant polypeptide is expressed, the nucleic acid may be bound to the recombinant polypeptide, thereby producing the nucleic acid-bound polypeptide.

To be more specific, when a polypeptide is expressed as a fusion polypeptide, with a nucleic acid-binding motif which is known to have a function of binding the nucleic acid to the polypeptide being included in the function of the polypeptide to be expressed, a polypeptide with the nucleic acid-binding motif is expressed, and the nucleic acid in the host is simultaneously bound to the recombinant polypeptide via the nucleic acid-binding motif, so that the nucleic acid-bound polypeptide can be produced. This nucleic acid-bound polypeptide can be purified thereafter.

Alternatively, the nucleic acid-bound polypeptide can be obtained by reconstituting the polypeptide by mixing the expressed polypeptide with the nucleic acid.

In connection with the above-mentioned nucleic acid-binding motif, various nucleic acid-binding motifs are known. For example, in J. of Virology, 64 3319-3330 (1990), there is reported a nucleic acid-binding motif which is present in HBc protein amino acid sequence of hepatitis B virus (HBV), and in Biochim. Biophys. Act, 950, 45-53 (1988), there is reported protamin, which is a nucleic acid-bound protein in mouse. These can also be employed in the present invention.

The nucleotide sequence and the amino acid sequence of the nucleic acid-binding motif of HBc are respectively shown in the sequence No. 1 and the sequence No. 2 in the sequence table attached to this specification; and the nucleotide sequence and the amino acid sequence of the mouse protamin are respectively shown in the sequence No. 17 and the sequence No. 18 in the sequence table attached to this specification

As mentioned above, when the protein or polypeptide conventionally produced by genetic engineering is used as the antigen to be fixed on the surface of the particles in the conventional agglutination immunoassay, it occasionally occurs that even though the polypeptide itself has reactivity with the antibody to be assayed and the antibody is in fact present in the test sample, no agglutination takes place.

In the present invention, however, this conventional problem is completely solved by use of the nucleic acid-bound polypeptide. Namely, when the nucleic acid-bound polypeptide of the present invention is used as the antigen to be fixed on the surface of particles for use in the agglutination immunoassay, the agglutination successfully takes place proportionally in accordance with the amount of the corresponding antibody in the test sample.

The nucleic acid-bound polypeptide of the present invention can be applied not only to the above-mentioned agglutination, but also to any conventional immunoassay such as ELISA (enzyme-linked immunosorbent assay).

Furthermore, the polypeptide antigen in a test sample can also be assayed by carrying out a competition reaction with the addition of a known amount of the nucleic acid-bound polypeptide to the test sample.

Conventionally, when a polypeptide is produced by genetic engineering, in many cases, the recombinant polypeptide is obtained as an insoluble fraction. Therefore, when the thus obtained polypeptide is used in practice, the polypeptide must be subjected to solubilization treatment. However, the polypeptide is often denatured in the course of the solubilization treatment, changing the antigenicity. Therefore it is preferable that the recombinant polypeptide be obtained as a soluble fraction.

In the method of producing the nucleic acid-bound polypeptide of the present invention, for example, a polypeptide is produced by genetic engineering, and the thus produced polypeptide is simultaneously caused to be bound to a nucleic acid in the host, whereby the nucleic acid-bound polypeptide is obtained as a soluble fraction.

Furthermore, as shown in the following examples, for example, when the polypeptide to be expressed is expressed as a fused polypeptide of a polypeptide and a nucleic acid-binding motif of HBc, the nucleic acid is bound to the nucleic acid-binding motif at the same time as the expression thereof, so that the nucleic acid-bound polypeptide is obtained in the soluble fraction.

Thus, there can be attained the method of producing the nucleic acid-bound polypeptide of the present invention, which comprises the steps of producing the recombinant polypeptide, binding the nucleic acid to the polypeptide to produce the nucleic acid-bound polypeptide as a soluble fraction, and purifying the nucleic acid-bound polypeptide from the soluble fraction. Other features of this invention will become apparent in the course of the following description of exemplary embodiments, which are given for illustration of the invention and are not intended to be limiting thereof.

### Reference Example 1

### [Expression of HCV Core Protein (1 - 120aa)]

A DNA fragment for coding the HCV core polypeptide with sequence ID. No. 3 in the attached sequence table was amplified by the PCR (Polymerase Chain Reaction) method, using as a template molecule a plasmid CKSC1150 with a DNA fragment including an HCV core region, and was then digested with a restriction endonuclease EcoRI and a restriction endonuclease BamHI.

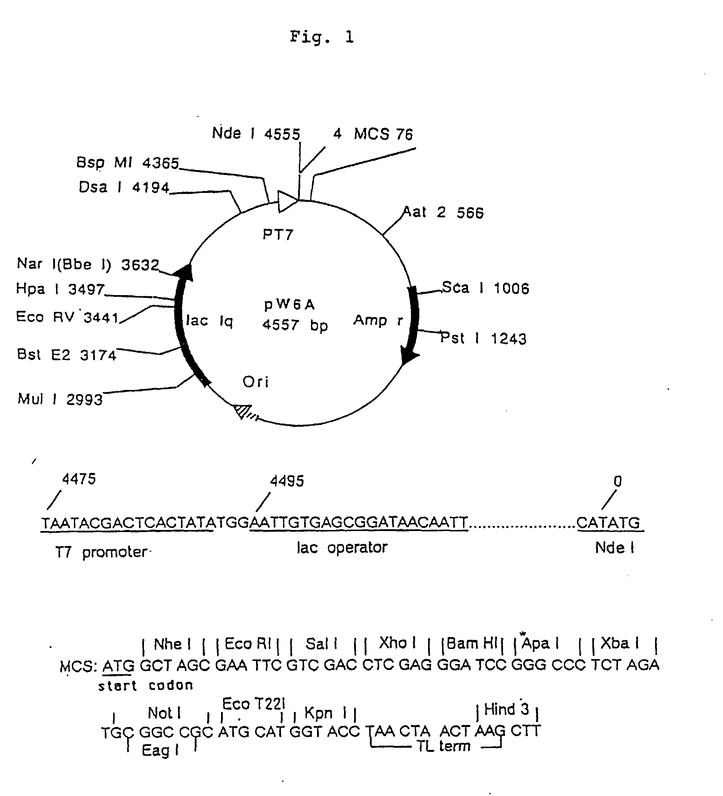

An HCV core region-including DNA fragment 370 bp was separated by 1% agarose gel electrophoresis. This DNA fragment was inserted into an EcoRI - BamHI site of an expression plasmid pW6A shown in Fig. 1, so that a plasmid pW6AHCV core 120 was prepared.

By use of this plasmid, *Escherichia coli* BL21 (DE3) (obtained from Brookhaven National Laboratory) was subjected to transformation, so that an ampicillin-resistant transformed *Escherichia coli* BL21 (DE3)/pW6AHCV core 120 for expressing HCV core polypeptide 120 was obtained, and an HCV core protein (1 - 120 aa) was expressed. Hereinafter, the thus expressed protein is referred to as "120". The nucleotide sequence of "120" and the amino acid sequence of "120" are respectively shown in Sequence ID. No. 3 and Sequence ID. No. 4 in the sequence table attached to this specification.

### Example 1

### [Preparation of Plasmid]

A DNA fragment for coding HCV core polypeptides 150 and 120 which are respectively shown with sequence ID. No. 5 and with sequence ID. No. 3 in the attached sequence table was amplified by the PCR (Polymerase Chain Reaction) method, using as a template molecule a plasmid CKSC1150 with a DNA fragment including an HCV core region being introduced, and was then digested with a restriction endonuclease EcoRI and a restriction endonuclease BamHI.

An HCV core region-including DNA fragment 470 bp and an HCV core region-including DNA fragment 370 bp were separated by 1% agarose gel electrophoresis. These DNA fragments were inserted into an EcoRI - BamHI site of the expression plasmid pW6A shown in Fig. 1, whereby a plasmid pW6AHCV core 150 and a plasmid pW6AHCV core 120 were prepared.

A DNA fragment for coding an HBc nucleic acid-binding motif shown with sequence ID. No. 1 in the sequence table attached to this specification was amplified by the PCR (Polymerase Chain Reaction) method, using as a template molecule a plasmid pHBV-11 (Nucleic Acids Res., 18, 4587 (1990)), and was then digested with the BamHI restriction endonuclease.

A DNA fragment 110bp including a nucleic acid-binding motif was separated by 2% agarose gel electrophoresis. This DNA fragment was inserted into an EcoRI - BamHI site of each of the above-mentioned plasmid pW6AHCV core 150 and plasmid pW6AHCV core 120.

By use of these plasmids, *Escherichia coli* BL21 (DE3) (obtained from Brookhaven National Laboratory) was subjected to transformation, so that an ampicillin-resistant transformed *Escherichia coli* BL21 (DE3)/pW6AHCV core 150NA and an ampicillin-resistant transformed *Escherichia coli* BL21 (DE3)/pW6AHCV core 120NA were obtained.

In this specification, the proteins to which the nucleic acid-binding motif is bound for expressing the above-mentioned transformed *Escherichia coli* BL21 (DE3)/pW6AHCV core 150NA and the above-mentioned transformed *Escherichia* coli BL21 (DE3)/pW6AHCV core 120NA are respectively referred to as "150NA" and "120NA".

The nucleotide sequence of "150NA" and the amino acid sequence of "150NA" are respectively shown in Sequence ID. No. 9 and Sequence ID. No. 10 in the sequence table attached to this specification; and the nucleotide sequence of "120NA" and the amino acid sequence of "120NA" are respectively shown in Sequence ID. No. 7 and Sequence ID. No. 8 in the sequence table attached to this specification.

### Example 2

### [Expression of Recombinant Protein (150NA and 120NA)]

Each of the transformed *Escherichia coli* BL21 (DE3)/pW6AHCV core 150 and the transformed *Escherlchia coli* BL21 (DE3)/pW6AHCV core 120 prepared in Example 1 was separately cultured overnight in 2 ml of an LB culture medium containing 50 µg/ml of ampicillin at 37°C.

After the optical density (OD) of each culture medium reached 0.6 to 0.8 with a light with a wavelength of 600 nm by preculture, expression induction was carried out with the addition of 0.5 mM IPTG (Isopropyl-β-D(-)-thiogalactopyranoside) thereto, and the cultivation was continued for another two hours.

1.5 ml of the *Escherichia coli* cultivation medium was centrifuged at 5000 rpm for 2 minutes, whereby the *Escherichia coli* was collected. The thus collected *Escherichia coli* was suspended in 100 µl of a buffer solution (10 mM tris-HCl, pH 8.0, 0.1 M NaCl, 1 mM EDTA), and was then subjected to ultrasonic disruption for 15 minutes, whereby the *Escherichia coli* was completely disrupted, whereby two test samples, namely an *Escherichia coli* test sample of *Escherichia coli* BL21 (DE3)/pW6AHCV core 150NA and an *Escherichia coli* test sample of *Escherichia coli* BL21 (DE3)/pW6AHCV core 120NA, were prepared.

8 µl of a three-fold concentrated SDS polyacrylamide buffer solution (0.15 M tris-HCl, pH 6.8, 6% SDS, 24% glycerol, 6 mM EDTA, 2% 2-mercaptoethanol, 0.003% bromophenol blue) was added to each of the above test samples separately. Each mixture was then stirred sufficiently and was subjected to SDS-polyacrylamide gel electrophoresis.

Western blotting was performed on a nitrocellulose filter, using each of the thus prepared test samples. After performing blocking using 1% BSA, each of the test samples was allowed to react with an HCV core antibody human serum which was diluted 200 times with a phosphoric acid buffer solution (10 mM phosphoric acid, pH 7.4, 0.15 M NaCl). Furthermore, a peroxydase enzyme labeled anti-human IgG rabbit polyclonal antibody (made by Daco Co., Ltd.) was then allowed to react therewith. After washing, 10 ml of a substrate coloring liquid (0.01 % aqueous solution of hydrogen peroxide, 0.6 mg/ml 4-chloro-1-naphthol) was added thereto, whereby each test sample was colored.

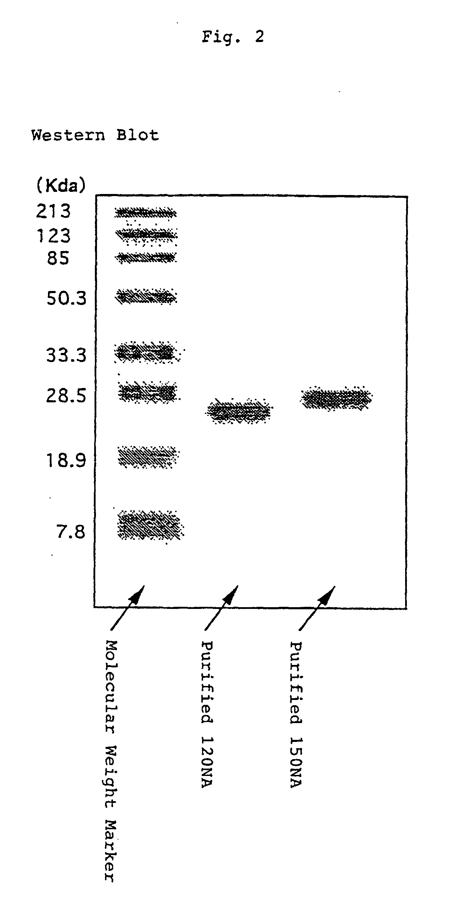

The results are shown in Fig. 2. As shown in Fig. 2, both the *Escherichia coli* test sample of *Escherichia coli* BL21 (DE3)/pW6AHCV core 150NA and the *Escherichia coli* test sample of *Escherichia coli* BL21 (DE3)/pW6AHCV core 120NA exhibited a positive reaction with the HCV core antibody human serum.

### Example 3

### [Purification of Soluble Nucleic Acid-bound 120NA Recombinant Protein (120(+))]

The *Escherichia coli* BL21 (DE3)/pW6AHCV core 120NA prepared in Example 1 was cultured overnight in an LB culture medium at 37°C. The optical density (OD) of the culture medium was adjusted by preculture so as to be about 0.7 when measured with light with a wavelength of 600 nm. Expression induction was then carried out with the addition of 0.5 mM IPTG thereto, and thereafter the cultivation was continued for two hours and 30 minutes.

The *Escherichia coli* cultivation medium was centrifuged, whereby the *Escherichia coli* was collected. To the thus collected *Escherichia coli*, 150 ml of a buffer solution (50 mM tris-HCl, pH 8.0, 20% ethanol, 0.2 M NaCl, 0.3% octylthioglucoside (hereinafter referred to as "OTG") were added, and the mixture was ice-cooled and subjected to ultrasonic disruption.

This mixture was then centrifuged, whereby a soluble fraction which contained therein a nucleic acid-bound 120NA (hereinafter referred to as "120NA(+)") was recovered.

A 50%-sucrose concentration buffer solution was prepared by adding sugar to a buffer solution (50 mM tris-HCl, pH 8.0, 20% ethanol) in such a manner that the concentration of sucrose in the buffer solution was 50%. In the same manner as mentioned above, a 30%-sucrose concentration buffer solution, and a 20%-sucrose concentration buffer solution were prepared.

These buffer solutions were overlaid in an ultracentrifuge tube in the direction from the bottom to the top portion of the tube in the order of the 50%-sucrose concentration buffer solution, the 30%-sucrose concentration buffer solution and the 20%-sucrose concentration buffer solution.

The 120NA(+) containing soluble fraction was overlaid on top of the overlaid buffer solutions in the ultracentrifuge tube, and was then subjected to a first sucrose density gradient ultracentrifugation at 10°C, with a centrifugal force of 100,000 g, for 12 hours, using a Beckman ultrasonic centrifuge.

The 120NA(+) was recovered in a portion with a sucrose concentration of about 30 to 40%.

The 120NA(+) containing fraction recovered by the first sucrose density gradient ultracentrifugation was purified by Superdex 200 (gel filtration column) (made by Pharmacia Co., Ltd.) which was equilibrated with a buffer solution (0.3 M NaCl, 0.1% myristyl sulfobetaine (Trademark "SB3-14" made by Sigma Co., Ltd.), whereby 120NA(+) with a molecular weight of about 700 to 1000 kDa was recovered.

A 50%-sucrose concentration buffer solution was prepared by adding sucrose to a buffer solution (50 mM tris-HCl, pH 8.0, 20% ethanol) in such a manner that the concentration of sucrose in the buffer solution was 50%. In the same manner as mentioned above, a 20%-sucrose concentration buffer solution was prepared.

These buffer solutions were overlaid in an ultracentrifuge tube in the direction from the bottom to the top portion of the tube in the order of the 50%-sucrose concentration buffer solution and the 20%-sucrose concentration buffer solution.

The above-mentioned 120NA(+) with a molecular weight of about 700 to 1000 kDa was overlaid on top of the overlaid buffer solutions in the ultracentrifuge tube, and was then subjected to a second sucrose density gradient ultracentrifugation at 10°C, with a centrifugal force of 100,000 g, for 12 hours, using the Beckman ultrasonic centrifuge, whereby the 120NA(+) was concentrated and purified.

### Reference Example 2

### [Purification of Insoluble 120NA]

The *Escherichia coli* BL21 (DE3)/pW6AHCV core 120NA prepared in Example 1 was cultured overnight in an LB culture medium at 37°C. The optical density (OD) of the culture medium was adjusted by preculture so as to have about 0.7 when measured with light with a wavelength of 600 nm. Expression induction was then carried out with the addition of 0.5 mM IPTG thereto, and the cultivation was continued for two hours and 30 minutes.

The *Escherichia coli* cultivation medium was then centrifuged, whereby the *Escherichia coli* was collected. To the thus collected *Escherichia coli*, 150 ml of a buffer solution (50 mM tris-HCl, pH 8.0, 20% ethanol, 0.2 M NaCl, 0.3% OTG) were added, and the mixture was ice-cooled and subjected to ultrasonic disruption.

This mixture was then centrifuged, whereby an insoluble 120NA fraction was obtained. The thus obtained insoluble 120NA fraction was made soluble by a buffer solution (6M urea, 50 mM glycine-NaOH, pH 11.7) and was then subjected to centrifugation, whereby a supernatant fraction was obtained.

The thus obtained supernatant fraction was subjected to ion exchange purification, using an SFF cationic ion exchange column (made by Pharmacia Co., Ltd.) which was equilibrated with a buffer solution (6M urea, 50 mM glycine-NaOH, pH 11.7), with sodium chloride elution.

The SFF eluted fraction was then purified, using Superdex 200 (gel filtration column) (made by Pharmacia Co., Ltd.) which was equilibrated with a buffer solution (6M urea-0.5M NaOH, 50 mM tris-HCl, pH 9.6). Thus, a purified 120NA was obtained in a portion with a molecular weight of about 22 kDa.

### Example 4

### [Confirmation of Properties of 120NA and 120NA (+)]

The OD 260/280 nm ratio of the 120NA(+) purified in Example 3 was measured. The result was that the OD 260/280 nm ratio of the 120NA(+) was about 2.0, which was greater than the OD 260/280 nm ratio of the 120NA. This indicated that at least the polypeptide and the nucleic acid coexist in the 120NA(+).

Furthermore, in the sucrose density gradient ultracentrifugation, the 120NA was mostly collected in the zero% sucrose concentration region, while the 120NA(+) was mostly collected in an about 30-40% sucrose concentration region. It is considered that this fact indicates that the density of the 120NA(+) is different from that of the 120NA.

The 120NA(+) was subjected to enzyme treatment, using DNase or RNase. When the 120NA(+) was subjected to enzyme treatment, using RNase, the nucleic acid contained in the 120NA(+) was decomposed in its entirety by the RNase. It is considered that this fact indicates that the constituent nucleic acid of the 120NA(+) is RNA.

The 120NA(+) was also subjected to isoelectric focusing. The isoelectric point of the 120NA(+) was present in a wide range of pI 3.5 to 5.0.

In sharp contrast to this, the isoelectric point of the 120NA purified in Reference Example 2 was pI 12.84, with a strong positive charge, which was significantly different from the isoelectric point of the 120NA(+).

Furthermore, the 120NA(+) was also subjected to Native electrophoresis, using a 3% agarose 3% polyacrylamide gel. From the fact that luminescence was observed at the time of Ethidium bromide stain of the 120NA(+), it was confirmed that the nucleic acid was contained in the 120NA(+).

The 120NA(+) was further subjected to Western blotting and Coomassie Brilliant Blue stain, using the same gel as used in the above-mentioned Ethidium Bromide stain. The result was that in the Western blotting, the reactivity of the 120NA(+) with an anti-HCV core antibody was observed at the same position as that of the portion made luminescent by the Ethidium Bromide stain; and in the Coomassie Brilliant Blue stain, the presence of the polypeptide was confirmed.

In sharp contrast to this, with respect to the 120A, the transfer of the 120NA into the gel was not confirmed in the Native electrophoresis even when the Western blotting and the Coomassie Brilliant Blue stain were carried out.

Thus, the properties of the 120NA(+) are entirely different from those of the 120NA with respect to the apparent molecular weight, the density, and the electric charge thereof, particularly because of the increase of the apparent molecular weight of the 120NA(+) due to the binding of the nucleic acid to the polypeptide in the 120NA(+), but there are no differences in the Western blotting and agglutination reactions between the two. From these facts, it is considered that the antigenicity is maintained in the 120NA(+).

### Reference Example 3

### [Expression of Lysine-fused 120 (120K10)]

In the same manner as in Example 1, pW6AHCV core 120 was subjected to such gene manipulation that 10 lysine residues were continuously fused to the C-terminus of pW6AHCV core 120, whereby pW6AHCV core 120K10 was prepared.

By use of this pW6AHCV core 120K10, *Escherichia coli* BL21 (DE3) was subjected to transformation, whereby an ampicillin-resistant transformed *Escherichia coli* BL21 (DE3)/pW6AHCV core K10 was obtained. Hereinafter, the protein expressed by this ampicillin-resistant transformed *Escherichia coli* BL21 (DE3)/pW6AHCV core K10 is referred to as 120K10.

The above transformed *Escherichia coli* BL21 (DE3)/pW6AHCV core K10 was cultured overnight in an LB culture medium at 37°C. The optical density (OD) of the culture medium was adjusted by preculture so as to have about 0.7 when measured with light with a wavelength of 600 nm. Expression induction was then carried out with the addition of 0.5 mM IPTG thereto, and the cultivation was continued for two hours and 30 minutes.

The *Escherichia coli* cultivation medium then was centrifuged, whereby the *Escherichia coli* was collected. To the thus collected *Escherichia coli*, 150 ml of a buffer solution (50 mM tris-HCl, pH 8.0, 20% ethanol, 0.2 M NaCl, 0.3% OTG) were added, and the mixture was ice-cooled and subjected to ultrasonic disruption.

This mixture was then centrifuged, whereby a soluble 120K10 fraction and an insoluble 120K10 fraction were separately obtained.

A 50%-sucrose concentration buffer solution was prepared by adding sucrose to a buffer solution (50 mM tris-HCl, pH 8.0, 20% ethanol) in such a manner that the concentration of sucrose in the buffer solution was 50%. In the same manner as mentioned above, a 30%-sucrose concentration buffer solution and a 20%-sucrose concentration buffer solution were prepared.

These buffer solutions were overlaid in an ultracentrifuge tube in the direction from the bottom to the top portion of the tube in the order of the 50%-sucrose concentration buffer solution, the 30%-sucrose concentration buffer solution and the 20%-sucrose concentration buffer solution.

The above-mentioned soluble 120K10 fraction was overlaid on top of the overlaid buffer solutions in the ultracentrifuge tube, and was then subjected to a sucrose density gradient ultracentrifugation at 10°C, with a centrifugal force of 100,000 g, for 12 hours, using the Beckman ultrasonic centrifuge. The 120K10 was not recovered in any of the 50%-sucrose concentration buffer solution, the 30%-sucrose concentration buffer solution and the 20%-sucrose concentration buffer solution, but was recovered on the top layer portion in the tube.

The above-mentioned insoluble 120K10 fraction was purified in the same manner as in Reference Example 2, using the SFF cationic ion exchange column (made by Pharmacia Co., Ltd.) and performing the gel filtration, whereby a purified 120K10 was recovered in a portion with a molecular weight of about 20 kDa.

### Reference Example 4

### [Assay of HCV Core Antigen Positive Serum]

The reactivity of each of HCV antibody positive serum 1 and HCV antibody positive serum 2 with a commercially available HCV antibody assay agent (Trademark "RIBA HCV 3.0 STRIP IMMUNOBLOT ASSAY" made by Chiron Co., Ltd.) was tested, using HCV antigen c100 (Amino acid Nos. 1569-1931), HCV antigen c33c (Amino acid Nos. 1192-1457), core antigen c22 (Amino acid Nos. 2-120) and NS5 (Amino acid Nos. 2054-2995). The result was that both HCV antibody positive serum 1 and HCV antibody positive serum 2 have antibodies in the entire antigen region including the core antigen region.

**TABLE 1**

| Reactivity Tests of Positive Serums | | | | | |
|---|---|---|---|---|---|
| | c100 | c33c | Core Antigen | NS5 | Judgement |
| Positive Serum 1 | 4+ | 4+ | 4+ | 4+ | Positive |
| | | | | | |
| Positive Serum 2 | 4+ | 4+ | 4+ | 4+ | Positive |

### Example 5

Each of the HCV antigens obtained in Reference Examples 1, 2, 3 and Example 3 was fixed on the surface of gelatin particles (made by Fujirebio Co., Ltd.) with a concentration of 10 mg/ml in a buffer solution (0.15M PBS, pH 7.1).

By use of HCV antibody positive serum 1 and HCV antibody positive serum 2 confirmed as having antibodies in the entire antigen region including the core antigen region in Reference Example 4, and a monoclonal antibody #2-7 obtained by subjecting HCV core antigen c22 to immunization, the immune reactivity of each of the above-mentioned HCV antigens fixed on the surface of gelatin particles was investigated.

25 µl of each HCV antigen-fixed gelatin particle and 25 µl of one of the above-mentioned HCV antibody positive serum 1 or HCV antibody positive serum 2, or 25 µl of the monoclonal antibody #2-7 were allowed to react in a microtiter plate (made by Fujirebio Co., Ltd.) for 2 hours, and agglutination images thereof were investigated. The results are shown in TABLE 2. In TABLE 2, the reactivity is shown with a dilution rate of 2ⁿ, and when a positive agglutination image was observed even when n was 4 or more in the dilution rate, the immune reactivity was judged as being "positive".

The monoclonal antibody #2-7 obtained by subjecting HCV core antigen c22 to immunization reacted with any HCV core antigen, but it was only with the 120NA(+) fixed gelatin particles that HCV antibody positive serum 1 and HCV antibody positive serum 2 reacted in the above-mentioned reactions.

**TABLE 2**

| Immune Reactivity Tests of HCV Core Antigens | | | |
|---|---|---|---|
| Name of Core Antigen | Positive Serum 1 | Positive Serum 2 | #2-7 |
| 120NA(+) | 6+ | 7 | 8 |
| 120NA | <3 | <3 | 7 |
| 120K10 | <3 | <3 | 6 |
| 120 | <3 | <3 | 4 |

### Example 6

### [Rearrangement of 120NA(+) from 120NA]

By use of the transformed *Escherichia coli* BL21 (DE3)/pW6AHCV core 120NA prepared in Example 1, HCV core 120NA was purified from an insoluble fraction thereof in the same manner as in Reference Example 2. The molecular weight of the purified HCV core 120NA was about 22 kDa, and the OD 260/280 nm ratio thereof was about 0.7.

To the HCV core 120NA (hereinafter referred to as 120NA), a cyclic plasmid DNA (4.7 Kbp) derived from pW6A, 6M urea and 20% sucrose were added, and 120NA was dialyzed against a buffer solution (50 mM tris-HCL, 0.15M NaCl, 20% sucrose), whereby 120NA was rearranged to 120NA(+).

The 120NA(+) which was obtained by the above-mentioned dialysis and rearrangement was purified, using Superdex 200 (gel filtration column) (made by Pharmacia Co., Ltd.), whereby the 120NA(+) was recovered in a portion with a molecular weight of 700 to 1000 KD.

A 50%-sucrose concentration buffer solution was prepared by adding sucrose to a buffer solution (50 mM tris-HCl, pH 8.0, 20% ethanol) in such a manner that the concentration of sucrose in the buffer solution was 50%. In the same manner as mentioned above, a 20%-sucrose concentration buffer solution was prepared.

These buffer solutions were overlaid in an ultracentrifuge tube in the direction from the bottom to the top portion of the tube in the order of the 50%-sucrose concentration buffer solution and the 20%-sucrose concentration buffer solution.

The above recovered 120NA(+) was overlaid on top of the overlaid buffer solutions in the ultracentrifuge tube, and was then subjected to a sucrose density gradient ultracentrifugation at 10°C, with a centrifugal force of 100,000 g, for 12 hours, using the Beckman ultrasonic centrifuge. The rearranged 120NA(+) was recovered in an about 40% to 50%-sucrose concentration portion of the buffer solution.

The OD 260/280 nm ratio of the 120NA before the rearrangement was about 0.7, and when the 120NA was rearranged to the 120NA(+), the OD 260/280 nm ratio thereof was changed from about 0.7 to about 1.7.

Furthermore, the above-mentioned rearranged 120NA(+) and the soluble 120NA(+) prepared in Example 3 have almost the same molecular weight after the gel filtration thereof, and also have almost the same specific weight thereof after the sucrose density gradient ultracentrifugation thereof. Thus, it is considered that these facts indicate that the above-mentioned rearrangement from the 120NA to the 120NA(+) was successfully conducted.

### Example 7

### [Construction of Transformed Escherichia coli BL21 (DE3)/pW6AHCV Core 120NA120 for Expressing 120-fused 120NA (120NA120)]

A DNA fragment for coding an HCV core polypeptide shown with sequence ID. No. 3 in the attached sequence table was amplified by the PCR method, using as a template molecule a plasmid CKSC1150 with a DNA fragment including an HCV core region being introduced, and was then digested with a restriction endonuclease NheI and a restriction endonuclease EcoRI.

An HCV core region-including DNA fragment 370 bp was separated by 1% agarose gel electrophoresis. This DNA fragment was inserted into an NheI - EcoRI site of the expression plasmid pW6A shown in Fig. 1, whereby a plasmid pW6AHCV core 120 (NheI/EcoRI) was prepared.

A DNA fragment for coding the HCV core polypeptide shown with sequence ID. No. 3 in the attached sequence table was amplified by the PCR method, using as a template molecule a plasmid CKSC1150, and was then digested with a restriction endonuclease EcoRI and a restriction endonuclease BamHI.

An HCV core region-including DNA fragment 370 bp was then separated by 1% agarose gel electrophoresis. This DNA fragment was inserted into an EcoRI - BamHI site of the plasmid pW6AHCV core 120 (NheI/EcoRI), whereby a plasmid pW6AHCV core 120-120 was prepared.

A DNA fragment for coding an HBc nucleic acid-binding motif with sequence ID. No. 1 in the attached sequence table was amplified by the PCR method, using as a template molecule a plasmid pHBV-11, and was then digested with a restriction endonuclease EcoRI.

A DNA fragment 110bp including the nucleic acid-binding motif was separated by 2% agarose gel electrophoresis. This DNA fragment was inserted into an EcoRI site of the above-mentioned plasmid pW6AHCV core 120-120.

By use of this plasmid, *Escherichia coli* BL21 (DE3) was subjected to transformation, so that an ampicillin-resistant transformed *Escherichia coli* BL21 (DE3)/pW6AHCV core 120NA120 for expressing 120-fused 120NA (hereinafter referred to as 120NA120) was obtained.

### Example 8

### [Purification of Insoluble 120NA120]

In the same manner as in Reference Example 2, the transformed *Escherichia coli* BL21 (DE3)/pW6AHCV core 120NA120 prepared in Example 7 was cultured overnight in an LB culture medium at 37°C. The optical density (OD) of the culture medium was adjusted by preculture so as to have about 0.7 when measured with light with a wavelength of 600 nm. Expression induction was then carried out with the addition of 0.5 mM IPTG thereto, and the cultivation was continued for two hours and 30 minutes.

The *Escherichia coli* cultivation medium was then centrifuged, whereby the *Escherichia coli* was collected. To the thus collected *Escherichia coli*, 150 ml of a buffer solution (50 mM tris-HCl, pH 8.0, 20% ethanol, 0.2 M NaCl, 0.3% OTG) were added, and the mixture was ice-cooled and subjected to ultrasonic disruption.

This mixture was then centrifuged, whereby an expressed 120NA120 was obtained as a soluble fraction as well as an insoluble fraction. The insoluble 120NA120 fraction was made soluble by a buffer solution (6M urea, 50 mM glycine-NaOH, pH 11.0) and was then subjected to centrifugation, whereby a supernatant fraction was obtained.

The thus obtained supernatant fraction was subjected to *ion* exchange purification, using an SFF cationic ion exchange column (made by Pharmacia Co., Ltd.) which was equilibrated with a buffer solution (6M urea-glycine-NaOH, pH 11.0), with sodium chloride elution. 120NA120 was recovered in an about 0.5M sodium chloride elution fraction.

The SFF eluted fraction was then purified, using Superdex 200 (gel filtration column)(made by Pharmacia Co., Ltd.) which was equilibrated with a buffer solution (6M urea-0.5M NaCl, 50 mM tris-HCl, pH 9.6). Thus, a purified 120NA120 was obtained in a portion with a molecular weight of about 40 kDa.

The nucleotide sequence and the amino acid sequence of the 120NA120 are respectively shown with Sequence ID. No. 11 and Sequence ID. No. 12 in the attached sequence table.

### Example 9

### [Purification of Soluble Nucleic Acid-bound 120NA120 (120NA120(+)]

In the same manner as in Example 3, the *Escherichia* *coli* BL21 (DE3)/pW6AHCV core 120NA120 prepared in Example 7 was cultured overnight in an LB culture medium at 37°C. The optical density (OD) of the culture medium was adjusted by preculture so as to be about 0.7 when measured with light with a wavelength of 600 nm. Expression induction was then carried out with the addition of 0.5 mM IPTG thereto, and thereafter the cultivation was continued for two hours and 30 minutes.

The *Escherichia coli* cultivation medium was centrifuged, whereby the *Escherichia coli* was collected. To the thus collected *Escherichia coli*, 150 ml of a buffer solution (50 mM tris-HCl, pH 8.0, 20% ethanol, 0.2 M NaCl, 0.3% OTG) were added, and the mixture was ice-cooled and subjected to ultrasonic disruption.

This mixture was then centrifuged, whereby a soluble nucleic acid-bound 120NA120 (hereinafter referred to as "120NA120(+)") was recovered.

A 50%-sucrose concentration buffer solution was prepared by adding sucrose to a buffer solution (50 mM tris-HCl, pH 8.0, 20% ethanol) in such a manner that the concentration of sucrose in the buffer solution was 50%. In the same manner as mentioned above, a 30%-sucrose concentration buffer solution, and a 20%-sucrose concentration buffer solution were prepared.

These buffer solutions were overlaid in an ultracentrifuge tube in the direction from the bottom to the top portion of the tube in the order of the 50%-sucrose concentration buffer solution, the 30%-sucrose concentration buffer solution and the 20%-sucrose concentration buffer solution.

The 120NA120(+) containing soluble fraction was overlaid on top of the overlaid buffer solutions in the ultracentrifuge tube, and was then subjected to a first sucrose density gradient ultracentrifugation at 10°C, with a centrifugal force of 100,000 g, for 12 hours, using a Beckman ultrasonic centrifuge.

The 120NA120(+) was recovered in a portion with a sucrose concentration of about 30 to 40%.

The 120NA120(+) containing fraction recovered by the first sucrose density gradient ultracentrifugation was purified by Superdex 200 (gel filtration column) (made by Pharmacia Co., Ltd.) which was equilibrated with a buffer solution (0.3 M NaCl, 0.3% OTG, 50 mM glycine-NaOH, pH 10.0), whereby 120NA120(+) with a molecular weight of about 700 to 1000 kDa was recovered.

A 50%-sucrose concentration buffer solution was prepared by adding sucrose to a buffer solution (50 mM tris-HCl, pH 8.0, 20% ethanol) in such a manner that the concentration of sucrose in the buffer solution was 50%. In the same manner as mentioned above, a 20%-sucrose concentration buffer solution was prepared.

These buffer solutions were overlaid in an ultracentrifuge tube in the direction from the bottom to the top portion of the tube in the order of the 50%-sucrose concentration buffer solution and the 20%-sucrose concentration buffer solution.

The above-mentioned 120NA120(+) with a molecular weight of about 700 to 1000 kDa was overlaid on top of the overlaid buffer solutions in the ultracentrifuge tube, and was then subjected to a second sucrose density gradient centrifugation at 10°C, with a centrifugal force of 100,000 g, for 12 hours, using the Beckman ultrasonic centrifuge, whereby the 120NA120(+) was concentrated and purified.

### Example 10

### [Rearrangement of 120NA120 to 120NA120(+)]

The OD 260/280 nm ratio of the 120NA120 purified in Example 9 was about 0.7.

To the purified 120NA120, there was added a purified DNA (about 1.3 to 0.7 Kbp)(made by Sigma Co., Ltd.), which was obtained form calf thymus and was subjected to sufficient cleavage by a restriction endonuclease Hae3. Furthermore, 6M urea, 20% sucrose and 1.0 M NaCl were added thereto.

This mixture was dialyzed against a buffer (50 mM tris-HCl, 0.3 M NaCl) at 4°C, whereby the 120NA120 was rearranged to a soluble 120NA120(+).

The soluble 120NA120(+) was purified by Superdex 200 (gel filtration column)(made by Pharmacia Co., Ltd.), whereby a purified 120NA120(+) was recovered in a portion with a molecular weight of about 700 to 1000 kDa. The OD 260/280 nm ratio of the thus recovered rearranged 120NA120(+) was about 1.8.

### Example 11

### [Construction of Transformed Escherichia coli BL21 (DE3)/pW6A47C2NA for Expressing Nucleic Acid-Binding TP47 (TP47C2NA)]

A DNA fragment encoding a 47 kDa antigen derived from TP (Treponema pallidum), with Sequence ID No. 13 in the attached sequence table, was amplified by the PCR method, using as a template molecule a plasmid pW6A47C2 with a DNA fragment including a TP 47 kDa antigen region being introduced, and was then digested with a restriction endonuclease EcoRI and a restriction endonuclease BamHI.

A TP 47 kDa antigen region-including DNA fragment 1.3 Kbp was separated by 1% agarose gel electrophoresis. This DNA fragment was inserted into an EcoRI-BamHI site of the expression plasmid pW6A shown in Fig. 1, whereby a plasmid pW6A47C2(EcoRI/BamHI) was prepared.

A DNA fragment for coding an HBc nucleic acid-binding motif with Sequence ID No. 1 in the attached sequence table was amplified by the PCR method, using as a template molecule a plasmid pHBV-11, and was then digested with a restriction endonuclease HamHI and a restriction endonuclease HindIII.

A nucleic acid-binding motif-containing DNA fragment 110 bp was then separated by 1% agarose gel electrophoresis. This DNA fragment was inserted into a BamHI-HindIII site of the above plasmid pW6A47C2 (EcoRI/BamHI).

By use of this plasmid, *Escherichia coli* BL21 (DE3) was subjected to transformation, so that an ampicillin-resistant transformed *Escherichia coli* BL21 (DE3)/pW6A47C2NA for expressing a nucleic acid-binding TP47 (hereinafter referred to as TP47C2NA) was obtained.

### Example 12

### [Purification of Insoluble TP47C2NA]

In the same manner as in Reference Example 2, the transformed *Escherichia coli* BL21 (DE3)/pW6ATP47C2NA prepared in Example 11 was cultured overnight in an LB culture medium at 37°C. The optical density (OD) of the culture medium was adjusted by preculture so as to have about 0.7 when measured with light with a wavelength of 600 nm. Expression induction was then carried out with the addition of 0.5 mM IPTG thereto, and the cultivation was continued for two hours and 30 minutes.

The *Escherichia coli* cultivation medium was then centrifuged, whereby the *Escherichia coli* was collected. To the thus collected *Escherichia coli*, 150 ml of a buffer solution (50 mM tris-HCl, pH 8.0, 20% ethanol) were added, and the mixture was ice-cooled and subjected to ultrasonic disruption.

This mixture was then centrifuged, whereby an expressed TP47C2NA was obtained as a soluble fraction as well as an insoluble fraction. The insoluble TP47C2NA fraction was made soluble by a buffer solution (6M urea, 50 mM tris-HCl, pH 8.0) and was then subjected to centrifugation, whereby a supernatant fraction was obtained.

The thus obtained supernatant fraction was subjected to ion exchange purification, using an SFF cationic ion exchange column (made by Pharmacia Co., Ltd.) which was equilibrated with a buffer solution (8M urea, sodium acetate, pH 6.0), with sodium chloride elution. TP47C2NA was recovered in an about 0.5M sodium chloride elution fraction.

The SFF eluted fraction was then purified, using Superdex 200 (gel filtration column)(made by Pharmacia Co., Ltd.) which was equilibrated with a buffer solution (6M urea-0.5M NaCl, 50 mM tris-HCl, pH 9.6). Thus, a purified TP47C2NA was obtained in a portion with a molecular weight of about 100 kDa.

The nucleotide sequence and the amino acid sequence of the TP47C2NA are respectively shown with Sequence ID No. 15 and Sequence ID No. 16 in the attached sequence table.

### Example 13

### [Purification of Soluble Nucleic Acid-bound TP47C2NA (TP47C2MA(+)]

In the same manner as in Example 3, the *Escherichia coli* BL21 (DE3)/pW6ATP47C2NA prepared in Example 11 was cultured overnight in an LB culture medium at 37°C. The optical density (OD) of the culture medium was adjusted by preculture so as to be about 0.7 when measured with light with a wavelength of 600 nm. Expression induction was then carried.out with the addition of 0.5 mM IPTG thereto, and thereafter the cultivation was continued for two hours and 30 minutes.

The *Escherichia coli* cultivation medium was centrifuged, whereby the *Escherichia coli* was collected. To the thus collected *Escherichia coli*, 150 ml of a buffer solution (50 mM tris-HCl, pH 8.0, 20% ethanol, 0.3% OTG) were added, and the mixture was ice-cooled and subjected to ultrasonic disruption.

This mixture was then centrifuged, whereby a soluble nucleic acid-bound TP47C2NA (hereinafter referred to as "TP47C2NA(+)") was recovered.

A 50%-sucrose concentration buffer solution was prepared by adding sucrose to a buffer solution (50 mM tris-HCl, pH 8.0, 20% ethanol) in such a manner that the concentration of sucrose in the buffer solution was 50%. In the same manner as mentioned above, a 30%-sucrose concentration buffer solution, and a 20%-sucrose concentration buffer solution were prepared.

These buffer solutions were overlaid in an ultracentrifuge tube in the direction from the bottom to the top portion of the tube in the order of the 50%-sucrose concentration buffer solution, the 30%-sucrose concentration buffer solution and the 20%-sucrose concentration buffer solution.

The TP47C2NA(+) containing soluble fraction was overlaid on top of the overlaid buffer solutions in the ultracentrifuge tube, and was then subjected to a first sucrose density gradient ultracentrifugation at 10°C, with a centrifugal force of 100,000 g, for 12 hours, using a Beckman ultrasonic centrifuge.

The TP47C2NA(+) was recovered in a portion with a sucrose concentration of about 30 to 45%.

The TP47C2NA(+) containing fraction recovered by the first sucrose density gradient ultracentrifugation was purified by Superdex 200 (gel filtration column) (made by Pharmacia Co., Ltd.) which was equilibrated with a buffer solution (0.3 M NaCl, 0.3% OTG, 50 mM glycine-NaOH, pH 10.0), whereby TP47C2NA(+) was recovered in a portion with a molecular weight of about 700 to 1000 kDa.

A 50%-sucrose concentration buffer solution was prepared by adding sucrose to a buffer solution (50 mM tris-HCl, pH 8.0, 20% ethanol) in such a manner that the concentration of sucrose in the buffer solution was 50%. In the same manner as mentioned above, a 20%-sucrose concentration buffer solution was prepared.

These buffer solutions were overlaid in an ultracentrifuge tube in the direction from the bottom to the top portion of the tube in the order of the 50%-sucrose concentration buffer solution and the 20%-sucrose concentration buffer solution.

The above-mentioned TP47C2NA(+) with a molecular weight of about 700 to 1000 kDa was overlaid on top of the overlaid buffer solutions in the ultracentrifuge tube, and was then subjected to a second sucrose density gradient centrifugation at 10°C, with a centrifugal force of 100,000 g, for 12 hours, using the Beckman ultrasonic centrifuge, whereby the TP47C2NA(+) was concentrated and purified.

### Example 14

### [Rearrangement of TP47C2NA to TP47C2NA(+)]

The OD 260/280 nm ratio of the TP47C2NA purified in Example 12 was about 0.6.

To the purified TP47C2NA, there was added a purified DNA (about 1.3 to 0.7 Kbp)(made by Sigma Co., Ltd.), which was obtained form calf thymus and was subjected to sufficient cleavage by a restriction endonuclease Hae3. Furthermore, 6M urea, 20% sucrose and 1.0 M NaCl were added thereto.

This mixture was dialyzed against a buffer (50 mM tris-HCl, 0.3 M NaCl) at 4°C, whereby the TP47C2NA was rearranged to a soluble TP47C2NA(+).

The soluble TP47C2NA(+) was purified by Superdex 200 (gel filtration column) (made by Pharmacia Co., Ltd.), whereby a purified 120NA120(+) was recovered in a portion with a molecular weight of about 700 to 1000 kDa. The OD 260/280 nm ratio of the thus recovered rearranged TP47C2NA(+) was about 1.8.

### Example 15

### [Construction of Transformed Escherichia coli BL21 (DE3)/pW6ACV Core 120prol for Expressing Mouse Protamine 1 fused 120 (120pro1)]

A DNA fragment for coding a mouse protamine 1 with Sequence ID No. 17 in the attached sequence table was isolated, and amplified by the PCR method, using as a template molecule a mouse protamine 1 cDNA, and was then digested with a restriction endonuclease EcoRI and a restriction endonuclease BamHI.

A mouse protamine 1 region-including DNA fragment 160 bp was separated by 1% agarose gel electrophoresis. This DNA fragment was inserted into an EcoRI-BamHI site of the plasmid pW6AHCV core 120 (NheI/EcoRI) prepared in Example 1.

By use of this plasmid, *Escherichia coli* BL21 (DE3) was subjected to transformation, so that an ampicillin-resistant transformed *Escherichia coli.* BL21 (DE3)/pW6ACV core 120prol for expressing a mouse protamine 1 fused 120 (hereinafter referred to as 120prol) was obtained.

### Example 16

### [Purification of 120pro1]

In the same manner as in Reference Example 2, the transformed *Escherichia coli* BL21 (DE3)/pW6AHCV core 120prol prepared in Example 15 was cultured overnight in an LB culture medium at 37°C. The optical density (OD) of the culture medium was adjusted by preculture so as to have about 0.7 when measured with light with a wavelength of 600 nm. Expression induction was then carried out with the addition of 0.5 mM IPTG thereto, and the cultivation was continued for 2 hours and 30 minutes.

The *Escherichia coli* cultivation medium was then centrifuged, whereby the *Escherichia coli* was collected. To the thus collected *Escherichia coli*, 150 ml of a buffer solution (50 mM tris-HCl, pH 8.0, 20% ethanol) were added, and the mixture was ice-cooled and subjected to ultrasonic disruption.

This mixture was then centrifuged, whereby an expressed 120prol was obtained as a soluble fraction as well as an insoluble fraction. The insoluble 120pro1 fraction was made soluble by a buffer solution (6M urea, 50 mM glycine-NaOH, pH 11.0) and was then subjected to centrifugation, whereby a supernatant fraction was obtained.

The thus obtained supernatant fraction was subjected to ion exchange purification, using an SFF cationic ion exchange column (made by Pharmacia Co., Ltd.) which was equilibrated with a buffer solution (6M urea-glycine-NaOH, pH 11.0), with sodium chloride elution. 120pro1 was recovered in an about 0.5M sodium chloride elution fraction.

The SFF eluted fraction was then purified, using Superdex 200 (gel filtration column)(made by Pharmacia Co., Ltd.) which was equilibrated with a buffer solution (6M urea-0.5M NaCl, 50 mM tris-HCl, pH 9.6). Thus, a purified 120prol was obtained in a portion with a molecular weight of about 22 kDa.

The nucleotide sequence and the amino acid sequence of the 120prol are respectively shown with Sequence ID No. 19 and Sequence ID No. 20 in the attached sequence table.

### Example 17

### [Rearrangement of 120pro1 to 120pro1(+)]

The OD 260/280 nm ratio of the 120prol purified in Example 16 was about 0.7.

To the purified 120prol, there was added a purified DNA (about 1.3 to 0.7 Kbp)(made by Sigma Co., Ltd.), which was obtained form calf thymus and was subjected to sufficient cleavage by a restriction endonuclease Hae3. Furthermore, 6M urea, 20% sucrose and 1.0 M NaCl were added thereto.

This mixture was dialyzed against a buffer (50 mM tris-HCl, 0.3M NaCl) at 4°C, whereby the 120prol was rearranged to a soluble 120pro1(+).

The soluble 120pro1(+) was purified by Superdex 200 (gel filtration column) (made by Pharmacia Co., Ltd.), whereby a purified 120pro1(+) was recovered in a portion with a molecular weight of about 700 to 1000 kDa. The OD 260/280 nm ratio of the thus recovered rearranged 120pro1(+) was about 1.7.

Thus, the present invention provides the nucleic acid-bound polypeptide with various properties of the polypeptide being changed, without changing the antigenicity thereof. The use of the nucleic acid-bound polypeptide of the present invention makes it possible to perform immunoassays which have been conventionally impossible.

Furthermore, according to the present invention, there is provided a method of recovering a genetic product in a soluble fraction, which has conventionally been recovered in an insoluble fraction.

## Claims

1. An immunoassay for assaying an antigen or an antibody corresponding to said antigen, wherein the antigen comprises a polypeptide, **characterized in that** said polypeptide comprises a nucleic acid-bound polypeptide, wherein said nucleic acid-bound polypeptide is a fusion polypeptide comprising a polypeptide with a nucleic acid-binding motif including the amino acid sequence shown in SEQ ID N° 2 whereto said nucleic acid is bound.

2. The immunoassay of claim 1, wherein said immunoassay is an agglutination assay, and said nucleic acid-bound polypeptide is fixed on the surface of particles.

## Patentansprüche

1. Immuntest zum Testen eines Antigens oder eines Antikörpers gegen das Antigen, wobei das Antigen ein Polypeptid umfasst, **dadurch gekennzeichnet, dass** das Polypeptid ein an Nucleinsäure gebundenes Polypeptid umfasst, wobei das an Nucleinsäure gebundene Polypeptid ein Fusionspolypeptid ist, umfassend ein Polypeptid mit einem Nucleinsäure bindenden Motiv, das die in SEQ ID Nr. 2 dargestellte Aminosäuresequenz einschließt, woran die Nucleinsäure gebunden ist.

2. Immuntest nach Anspruch 1, wobei der Immuntest ein Agglutinationstest ist, und das an Nucleinsäure gebundene Polypeptid auf der Oberfläche von Partikeln fixiert ist.

## Revendications

1. Immunotest pour tester un antigène ou un anticorps correspondant audit antigène, ledit antigène comprenant un polypeptide, **caractérisé en ce que** ledit polypeptide comprend un polypeptide lié à un acide nucléique, ledit polypepide lié à un acide nucléique étant un polypeptide de fusion comprenant un polypeptide ayant un motif de liaison d'acide nucléique incluant la séquence d'acides aminés SEQ ID N°2 auquel est lié ledit acide nucléique.

2. Immunotest selon la revendication 1, dans lequel ledit inmmunotest est un test d'agglutination et ledit polypeptide lié à un acide nucléique est fixé sur la surface de particules.
